# EUROPEAN PATENT SPECIFICATION

(11) **EP 2 897 473 B1**
(45) Date of publication and mention of the grant of the patent: **19.08.2020**
(21) Application number: 13789021.6
(22) Date of filing: 24.09.2013
(51) Int. Cl.: A21D 13/00, A23L 33/11

(54) **SERUM CHOLESTEROL LOWERING SNACK PRODUCT AND AN IMPROVED METHOD FOR LOWERING CHOLESTEROL**
SERUMCHOLESTERINSPIEGELSENKENDES GETRÄNK UND VERBESSERTES VERFAHREN ZUR SENKUNG DES CHOLESTERINSPIEGELS
PRODUIT DE GRIGNOTAGE DIMINUANT LE CHOLESTÉROL SÉRIQUE ET MÉTHODE AMÉLIORÉE POUR DIMINUER LE CHOLESTÉROL

(30) Priority: 24.09.2012 FI 20120313; 24.09.2012 US 201261704970 P
(43) Date of publication of application: 29.07.2015
(73) Proprietor: Raisio Nutrition Ltd, 21200 Raisio (FI)
(72) Inventor: KUUSISTO, Päivi, FI-21200 Raisio (FI); WESTER, Ingmar, FI-21200 Raisio (FI)
(86) International application number: PCT/FI2013/000033
(87) International publication number: WO 2014/044901

(56) References cited:
- WO-A1-2007/057511
- WO-A1-2007/057511
- US-A1- 2007 042 102
- US-A1- 2007 054 028
- US-A1- 2011 301 085
- US-A1- 2011 301 085

## Description

### Field of invention

The present invention relates to the field of healthy edible products, especially to serum cholesterol lowering non-drinkable snacks.

### Background

Elevated serum total and/or LDL (low density lipoprotein) cholesterol levels are one of the major risk factors for cardiovascular disease. One of the first steps in improving the serum cholesterol profile is changes in life style including changes in diet and exercise. Food products enriched with components having cholesterol lowering effect beyond normal nutrition have been commercially available for some time. Representative examples are food products with added plant sterols and/or plant stanols. Food products that contain added plant sterols and/or plant stanols are widely available and include for example margarines, spreads and dairy products such as yoghurts and drinks.

A recent meta-analysis (Demonty et al., J. Nutr. 139 (2009) 271-284) pointed out that simultaneous ingestion of a meal is required for obtaining the optimal cholesterol lowering efficacy of plant sterols and plant stanols. Thus, the consumers are usually advised to use the products containing plant sterols and/or plant stanols together with a meal for best cholesterol lowering efficacy. Doornbos et al. (Eur. J. Clin. Nutr. (2006) 60(3):325-33) studied the cholesterol lowering efficacy of single-dose plant sterol enriched yoghurt drinks at two different consumption conditions - when consumed with a meal (as a part of the lunch) or without a meal (before breakfast). The LDL cholesterol lowering efficacy of the drink was not optimal if consumed without a meal.

Currently consumers are advised to consume products containing plant sterols and/or plant stanols together with a meal to ensure the optimal cholesterol lowering efficacy. However, many consumers would prefer to consume the products containing plant sterols and/or plant stanols as a snack, without a meal. With the currently known formulations, the cholesterol lowering efficacy is not optimal if taken without a meal. The required composition of a formulation that would have ensured efficacy also as a snack is not known in the prior art. Plant sterols and plant stanols in esterified form have better cholesterol lowering efficacy, and would thus seem to be a preferred choice over free plant sterols and/or plant stanols also in such a snack formulation. Many consumers would also prefer to consume the products containing plant sterols and/or plant stanols only once a day, i.e. having the effective daily dose of plant sterols and/or plant stanols in a single portion (serving) of the snack product. US2011/301085 A1 relates to a therapeutical composition comprising a protein hydrolysate and a plant sterol, wherein the weight ratio of the plant sterol to the protein hydrolysate is from 1:0.02 to 1:150. Said composition can be used in a pharmaceutical, nutraceutical or food product for improving serum lipid profile.

Therefore, there is a need for a snack product containing plant sterols and/or plant stanols, wherein the snack product is suitable to be consumed without a simultaneous ingestion of a meal and still has an ensured serum LDL cholesterol lowering efficacy. Furthermore, there is a need for a snack product containing plant sterols and/or plant stanols wherein the effective daily dose of plant sterols and/or plant stanols preferably is in one portion of the snack product. There is also a need for an easy-to-use snack product that is convenient to carry with you without any need for cold storage.

### Summary of the invention

The invention relates to a snack portion with serum LDL cholesterol lowering effect. The snack portion comprises 0.8-15 g total plant sterol and plant stanol equivalents, of which equivalents 0.10-3.0 g are in free form, provided that 5.0-30 % of the equivalents are in free form, and the rest of the equivalents are in esterified form. The snack portion further comprises 3.0-15 g triglyceride fat and at least one additional edible ingredient. The water content of the snack portion is at most 15 % by weight.

The invention also relates to a snack product with serum LDL cholesterol lowering effect. The snack product comprises 1.0-20 w-% total plant sterol and plant stanol equivalents, of which equivalents 5.0-30 % are in free form, and the rest of the equivalents are in esterified form. The snack product further comprises 2.0-30 w-% triglyceride fat, at least one additional edible ingredient, and 0.1-15 w-% water.

The invention further relates to a pack containing the snack portion or the snack product disclosed above.

The invention still further relates to the snack product or the snack portion defined above for use in lowering serum LDL cholesterol, wherein the snack portion or the snack product is ingested without ingesting a meal.

### Detailed description of the invention

The present invention provides a nondrinkable snack product containing plant sterols and/or plant stanols, wherein the snack product is suitable to be consumed without a simultaneous ingestion of a meal and still have an ensured serum LDL cholesterol lowering efficacy. Furthermore, the current invention provides a snack product containing plant sterols and/or plant stanols wherein the effective daily dose of plant sterols and/or plant stanols preferably is in one portion of the snack product (i.e. snack portion).

The present invention discloses improved snack portions and snack products with an ensured serum LDL cholesterol lowering effect. The "ensured" serum LDL cholesterol lowering effect means that the serum LDL cholesterol lowering effect that is obtained when the snack portion or snack product according to the invention is consumed without a meal, is at least 70 % of the serum LDL cholesterol lowering effect of the same amount of total plant sterol and plant stanol equivalents consumed together with a meal. Preferably the serum LDL cholesterol lowering effect of the snack portion or snack product ingested without a meal is at least 75 %, more preferably at least 80 %, still more preferably at least 85 %, further more preferably at least 90 %, even further more preferably at least 95% and most preferably 100 % or at least 100 % of the effect of the same amount of total plant sterol and plant stanol equivalents taken together with a meal. The expected serum LDL cholesterol lowering efficacy with any daily intake of total plant sterol and/or plant stanol equivalents taken together with a meal can be calculated based on dose-response equations published by Demonty et al. (J. Nutr. 139 (2009) 271-284). Preferably the ensured serum LDL cholesterol lowering effect means that the serum LDL cholesterol lowering effect is maintained regardless of the consumption time of the snack portion or snack product in relation to ingesting a meal.

By "snack" is in this specification meant an edible product to be ingested between meals (breakfast, lunch or dinner).

It has surprisingly been found that a snack portion or snack product having an ensured LDL cholesterol lowering effect can be prepared by using combinations of plant sterols and/or plant stanols both in their free and esterified forms, triglyceride fat and at least one additional edible ingredient in certain amounts and/or ratios. To get the ensured LDL lowering effect, it is crucial to provide certain amounts and ratios of the named components. The recommended effective daily dose of plant sterols and/or plant stanols can be incorporated in a single portion of such a snack product. The snack portion or snack product according to the invention provides an easy and convenient way for the consumer to lower LDL cholesterol. The consumer gets the ensured LDL lowering effect by having a single portion (serving) of the product, at one consumption occasion, with recommended daily dose of plant sterols and/or plant stanols, without the requirement of having a meal simultaneously.

The invention relates to a snack portion with serum LDL cholesterol lowering effect, wherein the portion comprises
- 0.8-15 g, preferably 1.0-15 g, more preferably 1.5-12 g, still more preferably 1.8-10 g, even more preferably 2.0-8.0 g, further more preferably 2.2-7.0 g, and most preferably 2.5-6.0 g total plant sterol and plant stanol equivalents, of which equivalents
   a) 0.10-3.0 g, preferably 0.15-3.0 g, more preferably 0.18-2.5 g, still more preferably 0.20-2.0 g, even more preferably 0.20-1.5 g, still even more preferably 0.22-1.3 g, and most preferably 0.25-1.0 g are in free form, provided that 5.0-30 %, preferably 7.0-30 %, more preferably 8.0-30 %, still more preferably 10-30 %, even more preferably 13-30 %, further more preferably 16-30 %, most preferably 20-30 %; and typically at most 25 % are in free form, and
   b) the rest of the equivalents are in esterified form,
- 3.0-15 g, preferably 3.5-14 g, more preferably 4.0-13 g, still more preferably 4.5-12 g, even more preferably 5.0-12 g, further more preferably 5.5-12 g, still even more preferably 6.0-10 g, further even more preferably 7.0-10 g, and most preferably 8.0-10 g triglyceride fat,
- at least one additional edible ingredient, and
- water at most 15 %, preferably at most 10 %, and most preferably at most 5 % by weight of the portion.

The invention also relates to a snack product with serum LDL cholesterol lowering effect, wherein the product comprises
- 1.0-20 w-%, preferably 1.2-15 w-%, more preferably 1.5-12 w-% and most preferably 2.0-10 w-% total plant sterol and plant stanol equivalents, of which equivalents by weight
   a) 5.0-30 %, preferably 7.0-30 %, more preferably 8.0-30 %, still more preferably 10-30 %, even more preferably 13-30 %, further more preferably 16-30 %, most preferably 20-30 %; and typically at most 25 % are in free form, and
   b) the rest of the equivalents are in esterified form,
- 2.0-30 w-%, preferably 4.0-28 w-%, more preferably 6.0-25 w-%, even more preferably 8.0-25 w-%, still more preferably 10-25 w-%, further more preferably 12-25 %, even further more preferably 12-20 w-% and most preferably 15-20 w-% triglyceride fat,
- 0.1-15 w-%, preferably 0.1-10 w-%, and most preferably 0.1-5.0 w-% water, and
- 35-96.9 w-% (i.e. the rest) at least one additional edible ingredient.

The snack product preferably contains an amount of triglyceride fat of at least 3.0 g, preferably 3.0-15 g, more preferably 4.0-13 g, and most preferably 4.0-6.0 g per serving.

The snack product preferably contains an amount of protein of 1.5-30 g, preferably 3.0-20 g, more preferably at least 6.0 g, and most preferably at least 8.0 g per serving.

By "w-%" is here meant weight (i.e. weight per weight) percent.

By "snack product" is in this specification meant a food product meant to be consumed as snack. Non-limiting examples of food products that can be snack products or snack portions of the present invention are cookies (biscuits), bars, crackers, wafers, buns, cakes, salty and/or savory snacks (e.g. tortilla chips, bread sticks), candies. Preferred are cookies, bars and crackers, and most preferred are cookies. The cookie may also be sandwich-type with a filling between two pieces. The filling may contain at least part of the total plant sterol and plant stanol equivalents, triglyceride fat, and protein. These components may also be included at least partly as a coating e.g. on top of a cookie.

The snack portion or snack product according to the invention is aimed to have an ensured serum LDL cholesterol lowering effect also when used as a snack i.e. without ingesting a meal. This means that e.g. one portion can be consumed as a snack i.e. not accompanied by a meal at the same time. A portion can therefore suitably be consumed at a consumption occasion as defined herein and still give an ensured LDL cholesterol lowering effect.

By "consumption occasion" is in this specification meant the time used for ingesting the snack product or snack portion according to the invention (e.g. from 5 seconds to 30 minutes), as well as the time during which no meal is ingested before starting the ingestion of the snack product or portion (e.g. at least 30 minutes) and the time during which no meal is ingested after ending the ingestion of the snack product or portion (e.g. at least 30 minutes). The duration of the consumption occasion is therefore practically e.g. from 1 hour to 1.5 hours. The benefit obtained from the present invention is that the snack portion or product can be ingested at such a consumption occasion and still give an ensured LDL cholesterol lowering effect.

The snack product may of course also be consumed together with (i.e. including also immediately before or after) a meal or together with other snack products, but the aim of this invention is that the consumer obtains the ensured serum LDL cholesterol lowering effect even when consuming the snack product according to the present invention without a meal. The trader of the snack product or snack portion can therefore advise the consumer that the product or portion can be used as a snack effective in lowering serum LDL cholesterol.

By "snack portion" is meant the amount of the snack product meant to be consumed at one consumption occasion, i.e. the portion corresponds to a serving.

The portion size of the snack product is preferably 10-100 g, more preferably 20-60 g and most preferably 30-50 g. Typical sizes are at most 100 g, 75 g, 50 g and 40 g, and at least 10 g, 12 g and 15 g. The size of the snack portion is evidently the same. The size of the portion is suitable to be consumed at one consumption occasion.

The snack portion and the snack product according to the invention have an ensured serum LDL cholesterol lowering effect also when used as snacks. The consumer can therefore be advised that the snack product and the portion can be used as snacks. The snack portion and the snack product according to the invention have at least 70 % of the serum LDL cholesterol lowering effect when consumed without a meal compared to when the same amount of total plant sterol and plant stanol equivalents are consumed with a meal. Preferably, the portion as well as the product maintain the LDL cholesterol lowering efficacy regardless of the consumption time of the portion or product in relation to ingesting a meal. Therefore, the snack portion or snack product according to the invention is suitable to be ingested at a consumption occasion as defined herein.

By the term "edible ingredient" is here meant all components of the snack product or snack portion e.g. plant sterols and/or plant stanols, triglyceride fat, water and additional edible ingredients.

In this specification plant sterols include and preferably are 4-desmethyl sterols and 4-monomethyl sterols, and plant stanols include and preferably are 4-desmethyl stanols and 4-monomethyl stanols. Typical 4-desmethyl sterols are sitosterol, campesterol, stigmasterol, brassicasterol, 22-dehydro-brassicasterol and δ5-avenasterol. Typical stanols are sitostanol, campestanol and their 24-epimers. The term "plant sterols and/or plant stanols" includes all possible mixtures of sterols and/or stanols as well as any individual plant sterol and/or plant stanol.

The plant sterols and/or plant stanols used in this invention contain plant sterols and/or plant stanols in both free and esterified form. The plant sterols and/or plant stanols that are in esterified form, are esterified with a carboxylic acid or with a mixture of carboxylic acids. Examples of suitable carboxylic acids are fatty acids (having 2-24 carbon atoms, being saturated, monounsaturated or polyunsaturated, including also special fatty acids, such as EPA and DHA, and conjugated fatty acids, e.g. CLA). Preferably the plant sterols and/or plant stanols are esterified with fatty acids, most preferably with vegetable oil based fatty acids.

Plant stanol fatty acid ester and the effects thereof, as well as a suitable method for its preparation, are disclosed in US 6 174 560. Obviously also plant sterol esters can efficiently be produced by the production method disclosed in US 6 174 560. Alternatively fatty acid esters of plant sterols and/or plant stanols can be produced by any method disclosed in the art.

By "plant sterol equivalents" is meant the amount of plant sterols calculated as the amount of free plant sterols. This means that in case of plant sterol ester, only the plant sterol part of the molecule is calculated as a plant sterol equivalent and the acid part is ignored.

Correspondingly, by "plant stanol equivalents" is meant the amount of plant stanols calculated as the amount of free plant stanols. This means that in case of plant stanol ester, only the plant stanol part of the molecule is calculated as a plant stanol equivalent and the acid part is ignored.

By "total plant sterol and plant stanol equivalents" is thus meant the total amount of plant sterol equivalents and plant stanol equivalents. The "total plant sterol and plant stanol equivalents" therefore include plant sterols and plant stanols in both free and esterified form, however, expressed as the total amount of free plant sterols and free plant stanols. Thus, the equivalents may contain only plant sterols or only plant stanols or their mixtures.

Of the total plant sterol and plant stanol equivalents 5.0-30 %, preferably 7.0-30 %, more preferably 8.0-30 %, still more preferably 10-30 %, even more preferably 13-30 %, further more preferably 16-30 %, and most preferably 20-30 % are in free form (and most typically at most 25 % are in free form), and the rest of the equivalents are in esterified form.

The plant sterols and/or plant stanol composition used in the present invention may be obtained by stopping the esterification reaction when a desired degree of esterification has been achieved. Alternatively, in order to prepare the plant sterols and/or plant stanols, a suitable amount of free plant sterols and/or free plant stanols can be added to plant sterol ester and/or plant stanol ester which has been made e.g. utilising any conventional esterification method in which a high degree of esterification (typically at least 97 % by weight) can be obtained.

The plant sterols and/or plant stanols can be incorporated into the snack product or snack portion at any suitable production step in the preparation of the snack product or snack portion. The free plant sterols and/or plant stanols and the esterified plant sterols and/or plant stanols can also be added separately at any suitable production step. In that case the composition of plant sterols and/or plant stanols is at the latest formed within the final snack product or snack portion.

Most preferred in snack products or snack portions according to the present invention is a composition of plant sterols and/or plant stanols in which 50-100 % (by weight) of the total plant sterol and plant stanol equivalents are plant stanol equivalents. Preferably there is at least 70 %, more preferably at least 80 %, still more preferably at least 90 %, and most preferably at least 95 % by weight plant stanol equivalents, of the total plant sterol and plant stanol equivalents.

Still preferred in snack products and snack portions according to the present invention is a composition of plant sterols and/or plant stanols in which 50-100 % (by weight) of the plant sterols and/or plant stanols in free form is plant sterol. Preferably, of the total amount of free plant sterols and free plant stanols, at least 70 %, more preferably at least 80 %, still more preferably at least 90 %, and most preferably at least 95 % by weight are free plant sterols. It is, however, also possible, although not preferred, to have a composition in which 50-100 % by weight of the free plant sterols and/or plant stanols is free plant stanol.

The snack portion according to the invention contains plant sterols and/or plant stanols in an amount of
- 0.8-15 g, preferably 1.0-15 g, more preferably 1.5-12 g, still more preferably 1.8-10 g, even more preferably 2.0-8.0 g, further more preferably 2.2-7.0 g, and most preferably 2.5-6.0 g total plant sterol and plant stanol equivalents, of which equivalents
   a) 0.10-3.0 g, preferably 0.15-3.0 g, more preferably 0.18-2.5 g, still more preferably 0.20-2.0 g, even more preferably 0.20-1.5 g, still even more preferably 0.22-1.3 g, and most preferably 0.25-1.0 g are in free form, provided that 5.0-30 %, preferably 7.0-30 %; more preferably 8.0-30 %, still more preferably 10-30 %, even more preferably 13-30 %, further more preferably 16-30 %, most preferably 20-30 %; and typically at most 25 % are in free form, and
   b) the rest of the equivalents are in esterified form.

The snack product according to the invention contains plant sterols and/or plant stanols in an amount of
- 1.0-20 w-%, preferably 1.2-15 w-%, more preferably 1.5-12 w-% and most preferably 2.0-10 w-% total plant sterol and plant stanol equivalents, of which equivalents by weight
   a) 5.0-30 %, preferably 7.0-30 %, more preferably 8.0-30 %, still more preferably 10-30 %, even more preferably 13-30 %, further more preferably 16-30 %, most preferably 20-30 %; and typically at most 25 % are in free form, and
   b) the rest of the equivalents are in esterified form.

As used here, by "triglyceride fat" is meant edible fats and oils that consist mainly (at least 90, preferably at least 95 and most preferably at least 98 % by weight) of triacylglycerols. Triglyceride fat can be an intrinsic part of the other ingredients of the snack portion and snack product (i.e. triglyceride fat of cereals and nuts) or it can be separately added to the snack portion or snack product. Triglyceride fat can also be a mixture of different edible fats and/or oils. Typical examples of triglyceride fats are vegetable oils such as canola/rapeseed oil, soybean oil, sunflower oil, olive oil, corn oil, sesame seed oil, cottonseed oil, wheat germ oil, oat oil, linseed oil, peanut oil, camelina oil as well as triglyceride fat of dairy, fish and algal origin. The triglyceride fats can be naturally occurring or modified, for example hydrogenated, transesterified or contain structured triacylglycerols. Preferably the triglyceride fats are vegetable oils, most preferably vegetable oils containing omega-3 fatty acids. Preferably, at least part of the triglyceride fat is an intrinsic part of the other ingredients of the snack portion and snack product.

The snack portion according to the invention contains 3.0-15 g, preferably 3.5-14 g, more preferably 4.0-13 g, still more preferably 4.5-12 g, even more preferably 5.0-12 g, further more preferably 5.5-12 g, still even more preferably 6.0-10 g, further even more preferably 7.0-10 g, and most preferably 8.0-10 g triglyceride fat.

The snack product according to the present invention contains 2.0-30 w-%, preferably 4.0-28 w-%, more preferably 6.0-25 w-%, even more preferably 8.0-25 w-%, still more preferably 10-25 w-%, further more preferably 12-25 %, even further more preferably 12-20 w-%, and most preferably 15-20 w-% triglyceride fat.

The snack portion and snack product according to the present invention also contain at least one additional edible ingredient, such as a cereal (e.g. flour, flakes, bran), a dietary fiber (e.g. β-glucan, psyllium), an emulsifier, a stabiliser, a protein, a sweetening agent, a flavouring agent, a colouring agent, a preservative, and an ingredient with beneficial health effect, or mixtures of additional edible ingredients. The amount of the additional edible ingredient(s) in the snack product according to the invention is preferably from 35 to 96.9 % by weight. The more preferred amounts can be calculated as the rest, when adding together the amounts of all the other edible ingredients.

The snack portion and snack product of the present invention may contain protein as an additional edible ingredient. Examples of suitable proteins include dairy proteins, such as casein and whey protein; soy protein, various cereal proteins, such as wheat or rice proteins, pea protein, lupin protein, canola protein and mixtures of any of these. The protein can be an intrinsic part of the other ingredients of the snack portion and snack product (i.e. proteins in cereals and nuts) or can be separately added to the portion or product e.g. as protein concentrate or protein isolate.

The snack portion according to the invention may preferably contain 1.5-30 g, more preferably 2.0-25 g, still more preferably 3.0-20 g, even more preferably 4.0-15 g, further more preferably 8.0-15 g, and most preferably 9.0-15 g protein. Typically there is at least 5.0 g, more typically at least 6.0 g, still more typically at least 7.0 g, even more typically at least 8.0 g, and most typically at least 9.0 g protein per snack portion.

The protein content of the snack product is preferably from 1.0 to 50 % by weight, more preferably 3.0-45 %, still more preferably 4.0-40 %, even more preferably 6.0-35 %, further more preferably 7.0-35 %, still further more preferably 8.0-35 %, even more preferably 10-30 %, further more preferably 16-30 %, and most preferably 20-30 % by weight of the product.

The snack portion and snack product according to the present invention may contain various sweetening agents and flavouring agents as additional edible ingredient(s). As used here the term "sweetening agent" includes compounds used to increase the sweetness of the product. Non-limiting examples of these are e.g. sucrose and other sugars, sugar syrups, honey, sugar alcohols such as xylitol, maltitol, lactitol, sorbitol and erythritrol, non-carbohydrate sweeteners, such as aspartame, acesulfame-K, saccharin, cyclamates, sucralose and stevia. As used here the term "flavouring agent" means ingredients that bring flavour to the snack portion and snack product. Examples of these include various plant extracts; aromas such as vanilla; cocoa powder; chocolate chips; coffee; seeds; nuts; and preparations from berries, fruits and/or vegetables (e.g. dried or jams).

Non-limiting examples of ingredients with beneficial health effects are vitamins (e.g. C, D, E and K vitamins), minerals, fibres, antioxidants, polyphenols and probiotics.

The snack portion and snack product according to the present invention contain some water. The water content of the snack portion and snack product is from 0.1 to 15 % by weight. The water content of the portion and product according to the invention is typically at most 15 %, preferably at most 12 %, more preferably at most 10 %, even more preferably at most 8.0 % and most preferably at most 5.0 % by weight. Therefore, the snack portion and snack product are not drinks (nondrinkable snack portion and nondrinkable snack product).

The carbohydrate content of the snack portion and snack product of the present invention is 30-80 %, preferably 40-70 % and most preferably 55-65 % by weight.

The invention is further illustrated by the following highly preferred embodiments.

In one preferred embodiment, the snack portion comprises
- 1.8-10 g, preferably 2.0-8.0 g total plant sterol and plant stanol equivalents, of which equivalents
   a) 0.20-2.0 g, preferably 0.22 - 1.3 g, more preferably 0.25-1.0 g are in free form, provided that 10-30 %, preferably 13-30 %, more preferably 16-30 % and most preferably 20-30 % are in free form, and
   b) the rest of the equivalents are in esterified form,
- 4.0-13 g, more preferably 5.0-12 g, still more preferably 6.0-10 g, further more preferably 7.0-10 g and most preferably 8.0-10 g triglyceride fat,
- at least one additional edible ingredient comprising 1.5 - 30 g, preferably 3.0-20 g, more preferably 4.0-15 g, even more preferably 8.0-15 g and most preferably 9.0-15 g protein, and
- water at most 15 %, preferably at most 10 %, and most preferably at most 5 % by weight of the portion,
and the size of the snack portion is 10-100 g, preferably 20-60 g, more preferably 30-50 g.

In another preferred embodiment, the snack portion comprises
- 2.0 - 8.0 g total plant sterol and plant stanol equivalents, of which equivalents
   a) 0.25 - 1 g, are in free form, provided that 16-30 % and most preferably 20-30 % are in free form, and
   b) the rest of the equivalents are in esterified form,
- 5.0-12.0 g, more preferably 6.0-10 g, still more preferably 7.0-10 g and most preferably 8.0-10 g triglyceride fat,
- at least one additional edible ingredient comprising 3.0-20 g, more preferably 4.0-15 g, even more preferably 8.0-15 g, and most preferably 9.0-15 g protein, and
- water at most 15 %, preferably at most 10 %, and most preferably at most 5 % by weight of the portion,
and the size of the snack portion is 10-100 g, preferably 20-60 g, more preferably 30-50 g.

In still another preferred embodiment, the snack portion comprises
- 2.0 - 8.0 g total plant sterol and plant stanol equivalents, of which equivalents
   a) 0.25 - 1 g are in free form, provided that 16-30 % and most preferably 20-30 % are in free form, and
   b) the rest of the equivalents are in esterified form,
- 5.0-12.0 g, more preferably 6.0-10 g, still more preferably 7.0-10 g and most preferably 8.0-10 g triglyceride fat,
- at least one additional edible ingredient comprising at least 8.0 g, more preferably at least 9.0 g protein, and
- water at most 15 %, preferably at most 10 %, and most preferably at most 5 % by weight of the portion,
and the size of the snack portion is 10-100 g, preferably 20 - 60 g. more preferably 30-50 g.

In a further another preferred embodiment, the snack portion comprises
- 0.8-15 g, preferably 1.0-15 g, more preferably 1.5-12 g, still more preferably 1.8-10 g, even more preferably 2.0-8.0 g, further more preferably 2.2-7.0 g, and most preferably 2.5-6.0 g total plant sterol and plant stanol equivalents, of which equivalents 50-100 %, preferably at least 70 % by weight are plant stanol equivalents, and of which equivalents
   a) 0.10-3.0 g, preferably 0.15-3.0 g, more preferably 0.18-2.5 g, still more preferably 0.20-2.0 g, even more preferably 0.20-1.5 g, still even more preferably 0.22-1.3 g, and most preferably 0.25-1.0 g are in free form, provided that 5.0-30 %, preferably 7.0-30 %, more preferably 8.0-30 %, still more preferably 10-30 %, even more preferably 13-30 %, further more preferably 16-30 %, most preferably 20-30 % are in free form, and that 50-100 %, preferably at least 70 % by weight of the equivalents in free form are plant sterols, and
   b) the rest of the equivalents are in esterified form,
- 3.0-15 g, preferably 3.5-14 g, more preferably 4.0-13 g, still more preferably 4.5-12 g, even more preferably 5.0-12 g, further more preferably 5.5-12 g, even further more preferably 6.0-10 g, still even further more preferably 7.0-10 g and most preferably 8.0-10 g triglyceride fat"
- at least one additional edible ingredient, and
- water at most 15 %, preferably at most 10 %, and most preferably at most 5 % by weight of the portion.

In a further another preferred embodiment, the snack portion comprises
- 1.8-10 g, preferably 2.0-8.0 g total plant sterol and plant stanol equivalents, of which equivalents 50-100 %, preferably at least 70 % by weight are plant stanol equivalents, and of which equivalents
   a) 0.20-2.0 g, preferably 0.22 - 1.3 g, more preferably 0.25-1.0 g are in free form, provided that 10-30 %, preferably 16-30 % and most preferably 20-30 % are in free form, and that 50-100 %, preferably at least 70 % by weight of the equivalents in free form are plant sterols, and
   b) the rest of the equivalents are in esterified form
- 3.0-15 g, more preferably 4.0-13 g, still more preferably 5.0-12 g, further more preferably 6.0-10 g, still further more preferably 7.0-10 and most preferably 8.0-10 g triglyceride fat,
- at least one additional edible ingredient comprising 1.5 - 30 g, preferably 3.0-20 g, more preferably 4.0-15 g, even more preferably 8.0-15 g, and most preferably 9.0-15 g protein, and
- water at most 15 %, preferably at most 10 %, and most preferably at most 5 % by weight of the portion,
and the size of the snack portion is 10-100 g, preferably 20-60 g, more preferably 30-50 g.

In one preferred embodiment, the snack product comprises
- 1.5-12 w-%, preferably 2.0-10 w-% total plant sterol and plant stanol equivalents, of which equivalents by weight
   a) 10-30 %, preferably 13-30 %, more preferably 16-30 %, most preferably 20-30 % are in free form, and
   b) the rest of the equivalents are in esterified form,
- 10-25 w-%, preferably 12-25 %, more preferably 12-20 w-% and most preferably 15-20 w-% triglyceride fat,
- 0.1-15 w-%, preferably 0.1-10 w-%, and most preferably 0.1-5.0 w-% water, and
- at least one additional edible ingredient as the rest of 100 w-%, the ingredient comprising 6.0-35 w-%, preferably 7.0-35 w-%, more preferably 8.0-35 w-%, still more preferably 10-30 w-%, further more preferably 16-30 w-% and most preferably 20-30 w-% protein of the snack product.

In another preferred embodiment, the snack product comprises
- 2.0-10 w-% total plant sterol and plant stanol equivalents, of which equivalents by weight
   a) 16-30 %, most preferably 20-30 % are in free form, and
   b) the rest of the equivalents are in esterified form,
- 12-25 %, and more preferably 12-20 w-%, and most preferably 15-20 w-% triglyceride fat,
- 0.1-15 w-%, preferably 0.1-10 w-%, and most preferably 0.1-5.0 w-% water, and
- at least one additional edible ingredient as the rest of 100 w-%, the ingredient comprising 8.0-35 w-% more preferably 10-30 w-%, still more preferably 16-30 w-% and most preferably 20-30 w-% protein of the snack product.

In still another preferred embodiment, the snack product comprises
- 1.0-20 w-%, preferably 1.2-15 w-%, more preferably 1.5-12 w-% and most preferably 2.0-10 w-% total plant sterol and plant stanol equivalents, of which equivalents 50-100 %, preferably at least 70 % by weight are plant stanol equivalents, and of which equivalents by weight
   a) 5.0-30 %, preferably 7.0-30 %, more preferably 8.0-30 %, still more preferably 10-30 %, even more preferably 13-30 %, further more preferably 16-30 %, most preferably 20-30 % are in free form, provided that 50-100 %, preferably at least 70 % by weight of the equivalents in free form are plant sterols, and
   b) the rest of the equivalents are in esterified form,
- 2.0-30 w-%, preferably 4.0-28 w-%, more preferably 6.0-25 w-%, even more preferably 8.0-25 w-%, still more preferably 10-25 w-%, further more preferably 12-25 %, still further more preferably 12-20 w-% and most preferably 15-20 w-% triglyceride fat,
- 0.1-15 w-%, preferably 0.1-10 w-%, and most preferably 0.1-5.0 w-% water, and
- 35-96.9 w-% at least one additional edible ingredient.

In still another preferred embodiment, the snack product comprises
- 1.5-12 w-%, preferably 2.0-10 w-% total plant sterol and plant stanol equivalents, of which equivalents 50-100 %, preferably at least 70 % by weight are plant stanol equivalents, and of which equivalents by weight
   a) 10-30 %, preferably 13-30 %, more preferably 16-30 %, most preferably 20-30 % are in free form, provided that 50-100 %, preferably at least 70 % by weight of the equivalents in free form are plant sterols, and
   b) the rest of the equivalents are in esterified form,
- 10-25 w-%, preferably 12-25 %, more preferably 12-20 w-% and most preferably 15-20 w-% triglyceride fat,
- 0.1-15 w-%, preferably 0.1-10 w-%, and most preferably 0.1-5.0 w-% water, and
- at least one additional edible ingredient as the rest of 100 w-%, the ingredient comprising 6.0-35 w-%, preferably 7.0-35 w-%, more preferably 8.0-35 w-%, still more preferably 10-30 w-%, further more preferably 16-30 w-% and most preferably 20-30 w-% protein of the snack product.

Within above disclosed highly preferred embodiments and also in the detailed description before the highly preferred embodiments or in the claims, presented preferred alternatives, amounts and ratios are all meant to be combinable with each other into preferred snack portions or snack products according to the present invention.

The invention further relates to a pack containing the snack portion or snack product with serum LDL cholesterol lowering effect as disclosed above.

By "pack" is here meant the physical protection within which the snack portion or snack product is enclosed. The pack may contain one or more snack portions, but most preferably it contains one snack portion. The snack portion may consist of one, two or several pieces, but preferably it is in one piece.

The pack may further deliver the advice to the consumer that the snack portion has an ensured serum LDL cholesterol lowering effect also when used as a snack. This means that the snack portion has at least 70 % of the serum LDL cholesterol lowering effect when consumed without a meal compared to when the same amount of total plant sterol and plant stanol equivalents are consumed with a meal. The snack portion preferably maintains the LDL cholesterol lowering efficacy regardless of the consumption time thereof in relation to ingesting a meal. Most preferably the pack according to the invention can deliver the advice to the consumer that the snack portion can be used as a snack with effective serum cholesterol lowering effect.

The description further discloses a method for lowering serum LDL cholesterol in a subject in need thereof, wherein the subject ingests a snack product or snack portion according to the invention, preferably once a day, without ingesting a meal. This means that the method of lowering LDL cholesterol is improved in that way that the snack product or snack portion can be consumed without ingesting a meal at the same time. This means that the snack product or snack portion can be ingested as a snack, i.e. not accompanied by a meal at the same time of consumption. The method therefore enables an ensured lowering of serum LDL cholesterol without ingesting a meal at the same consumption occasion, e.g. not ingesting a meal within 30 minutes before starting and 30 minutes after ending the consumption of the snack product or snack portion. The aim of the method is that the subject obtains the ensured serum LDL cholesterol lowering effect even when using the snack product or snack portion according to the present invention without a meal. Thus, the invention further relates to the snack product or the snack portion for use in lowering serum LDL cholesterol, wherein the snack portion or the snack product is ingested without ingesting a meal.

The analysis of the fatty components of the snack portion and snack product is performed in a known manner. The content of triglyceride fat, total plant sterol and plant stanol equivalents (including both esterified and free plant sterols and plant stanols, as defined above), and free plant sterols and plant stanols can be determined in the following way: The content of total plant sterol and plant stanol equivalents is analysed by saponifying the sample with alcoholic potassium hydroxide, extracting the unsaponifiable matter with hexane, and analysing as silyl ether derivatives by a gas chromatographic method. Free plant sterols and plant stanols are analysed by using solid phase extraction and gas chromatographic analysis. The amount of plant sterol and plant stanol equivalents in esterified form is calculated by subtracting the amount of free plant sterols and plant stanols from the amount of total plant sterol and plant stanol equivalents. The amount of plant sterol ester and plant stanol ester is calculated by multiplying the amount of plant sterol and plant stanol equivalents in esterified form by 1.67. The crude fat content is analysed by acid hydrolysis and Büchi extraction. From the crude fat content the amount of free plant sterols and plant stanols and the amount of plant sterol ester and plant stanol ester are subtracted to obtain the content of triglyceride fat.

The invention is further illustrated by the following non-limiting examples.

### Example 1

**Snack cookie**

| | % by weight |
|---|---|
| oat flakes | 18.0 |
| wheat flour | 21.5 |
| oat bran | 13.0 |
| sugar | 10.0 |
| dried apricot, apple | 6.0 |
| starch | 5.85 |
| polydextrose | 2.0 |
| baking powder | 0.7 |
| salt | 0.45 |
| flavour | 0.5 |
| water | 0.5 |
| rapeseed oil | 11.0 |
| plant stanol/sterol composition* | 10.5 |

| | |
|---|---|
| * in the composition 90 % of the total equivalents were esterified plant stanols and 10 % of the equivalents were free plant sterols | |

The dough was prepared by mixing all ingredients. Cookies were formed, baked and cooled. The weight of a cookie was 30 g (a portion size). In a portion there was 2.0 g of total plant sterol and plant stanol equivalents; 0.20 g of them were free plant sterols and 1.8 g were plant stanols esterified with rapeseed oil fatty acids. The amount of triglyceride fat was 4.2 g and of protein 2.2.g in the snack portion.

The snack cookie contained 6.7 w-% total plant sterol and plant stanol equivalents, 14.0 w-% triglyceride fat and 7.3 w-% protein.

### Example 2

**Snack cereal bar**

| | % by weight |
|---|---|
| oat flakes | 32.0 |
| oat bran | 8.0 |
| corn syrup | 12.0 |
| brown sugar | 2.0 |
| concentrated apple juice | 5.5 |
| apple | 2.0 |
| raisin | 3.0 |
| vegetable fat | 12.0 |
| soy protein isolate (95 % by weight protein) | 9.0 |
| salt | 0.5 |
| flavouring | 0.5 |
| lecithin (not deoiled) | 0.5 |
| plant stanol/sterol composition * | 13.0 |

| | |
|---|---|
| * in the composition 80 % of the total equivalents were esterified plant stanols and 20 % of the equivalents were free plant sterols | |

The weight of a bar was 40 g (a portion). There was 3.4 g of total plant sterol and plant stanol equivalents, 0.7 g of them were free plant sterols and 2.7 g were plant stanols esterified with rapeseed oil fatty acids. The amount of triglyceride fat was 6.2 g and of protein 5.8 g in the snack portion.

The snack cereal bar contained 8.5 w-% total plant sterol and plant stanol equivalents, 15.5 w-% triglyceride fat and 14.5 w-% protein.

### Example 3

**Snack cookie**

| | % by weight |
|---|---|
| oat flakes | 30 |
| wheat flour | 16 |
| rye flakes | 3 |
| rapeseed oil | 10 |
| sugar | 11 |
| soy protein isolate (95% protein) | 10 |
| dried fruits | 4 |
| water | 5 |
| salt | 0.4 |
| baking powder | 0.6 |
| plant stanol/sterol composition * | 10 |

| | |
|---|---|
| * in the composition 80 % of the total equivalents were esterified plant stanols and 20 % of the equivalents were free plant sterols | |

The weight of the cookie was 30 g. There was 2.0 g of total plant sterol and plant stanol equivalents, 0.4 g of them were free plant sterols and 1.6 g were plant stanols esterified with rapeseed oil fatty acids. The amount of triglyceride fat was 3.8 g and of protein 4.8 g in the snack portion.

The snack cookie contained 6.5 w-% total plant sterol and plant stanol equivalents, 12.7 w-% triglyceride fat and 16.0 w-% protein.

### Example 4

**Snack bar with high protein content**

| | |
|---|---|
| | % by weight |
| oat flakes | 35.0 |
| wheat flakes | 8.0 |
| rapeseed oil | 10.8 |
| vegetable fat | 4.0 |
| corn syrup | 10.0 |
| concentrated apple juice | 4.0 |
| soy protein isolate (95% protein) | 15.2 |
| dried fruits | 4.0 |
| salt | 0.2 |
| flavoring | 0.3 |
| emulsifier | 0.2 |
| plant stanol/sterol composition * | 8.3 |

| | |
|---|---|
| * in the composition 75 % of the total equivalents were esterified plant stanols and 25 % of the equivalents were free plant sterols | |

The weight of the protein snack bar was 40 g. There was 2.2 g of total plant sterol and plant stanol equivalents, 0.55 g of them were free plant sterols and 1.65 g were plant stanols esterified with rapeseed oil fatty acids. The amount of triglyceride fat was 7.0 g and of protein 8.2 g in the snack portion.

The snack bar contained 5.5 w-% total plant sterol and plant stanol equivalents, 17.5 w-% triglyceride fat and 20.5 w-% protein.

### Example 5

**Snack cookie**

| | % by weight |
|---|---|
| Sugar | 16.0 |
| Salt | 0.2 |
| Baking powder | 0.2 |
| Sodium bicarbonate | 0.2 |
| Vegetable fat | 13.5 |
| Water | 4.0 |
| Honey | 4.5 |
| Oat bran | 10.5 |
| Wheaf flour | 6.5 |
| Plant stanol/sterol composition* | 10.2 |
| Starch | 19.9 |
| Oat flakes | 9.4 |
| Soy protein isolate (95 % protein) | 4.9 |

| | |
|---|---|
| * in the composition 80 % of the total equivalents were esterified plant stanols and 20 % of the equivalents were free plant sterols | |

The weight of the snack cookie was 30 g. There was 2.0 g of total plant sterol and plant stanol equivalents, 0.40 g of them were free plant sterols and 1.60 g were plant stanols esterified with rapeseed oil fatty acids. The amount of triglyceride fat was 4.5 g and of protein 2.6 g in the snack portion.

The snack bar contained 6.7 w-% total plant sterol and plant stanol equivalents, 15.1 w-% triglyceride fat and 8.7 w-% protein.

## Claims

1. A snack portion with serum LDL cholesterol lowering effect, wherein the snack portion comprises
- 0.8-15 g, preferably 1.0-15 g, more preferably 1.5-12 g, still more preferably 1.8-10 g, even more preferably 2.0-8.0 g, further more preferably 2.2-7.0 g, and most preferably 2.5-6.0 g total plant sterol and plant stanol equivalents, of which equivalents
a) 0.10-3.0 g, preferably 0.15-3.0 g, more preferably 0.18-2.5 g, still more preferably 0.20-2.0 g, even more preferably 0.20-1.5 g, still even more preferably 0.22-1.3 g, and most preferably 0.25-1.0 g are in free form, provided that 5.0-30 %, preferably 7.0-30 %, more preferably 8.0-30 %, still more preferably 10-30 %, even more preferably 13-30 %, further more preferably 16-30 %, most preferably 20-30 % are in free form, and
b) the rest of the equivalents are in esterified form,
- 3.0-15 g, preferably 3.5-14 g, more preferably 4.0-13 g, still more preferably 4.5-12 g, even more preferably 5.0-12 g, further more preferably 5.5-12 g, still even more preferably 6.0-10 g, further even more preferably 7.0-10 g, and most preferably 8.0-10 g triglyceride fat,
- at least one additional edible ingredient, and
- water at most 15 %, preferably at most 10 %, and most preferably at most 5 % by weight of the portion.

2. The snack portion according to claim 1, wherein the additional edible ingredients comprise protein in an amount of 1.5-30 g, more preferably 2.0-25 g, still more preferably 3.0-20 g, even more preferably 4.0-15 g, further more preferably 8.0-15 g, and most preferably 9.0-15 g.

3. The snack portion according to claim 1 or 2, wherein it comprises
- 1.8-10 g, preferably 2.0-8.0 g total plant sterol and plant stanol equivalents, of which equivalents
a) 0.20-2.0 g, preferably 0.22 - 1.3 g, more preferably 0.25-1.0 g are in free form, provided that 10-30 %, preferably 13-30 %, more preferably 16-30 % and most preferably 20-30 % are in free form, and
b) the rest of the equivalents are in esterified form,
- 4.0-13 g, more preferably 5.0-12 g, still more preferably 6.0-10 g, further more preferably 7.0-10 g and most preferably 8.0-10 g triglyceride fat,
- at least one additional edible ingredient comprising 1.5 - 30 g, preferably 3.0-20 g, more preferably 4.0-15 g, even more preferably 8.0-15 g and most preferably 9.0-15 g protein, and
- water at most 15 %, preferably at most 10 %, and most preferably at most 5 % by weight of the portion,
and the size of the snack portion is 10-100 g, preferably 20-60 g, more preferably 30-50 g.

4. The snack portion according to claim 1, wherein the snack portion comprises
- 0.8-15 g, preferably 1.0-15 g, more preferably 1.5-12 g, still more preferably 1.8-10 g, even more preferably 2.0-8.0 g, further more preferably 2.2-7.0 g, and most preferably 2.5-6.0 g total plant sterol and plant stanol equivalents, of which equivalents 50-100 %, preferably at least 70 % by weight are plant stanol equivalents, and of which equivalents
a) 0.10-3.0 g, preferably 0.15-3.0 g, more preferably 0.18-2.5 g, still more preferably 0.20-2.0 g, even more preferably 0.20-1.5 g, still even more preferably 0.22-1.3 g, and most preferably 0.25-1.0 g are in free form, provided that 5.0-30 %, preferably 7.0-30 %, more preferably 8.0-30 %, still more preferably 10-30 %, even more preferably 13-30 %, further more preferably 16-30 %, most preferably 20-30 % are in free form, and that 50-100 %, preferably at least 70 % by weight of the equivalents in free form are plant sterols, and
b) the rest of the equivalents are in esterified form,
- 3.0-15 g, preferably 3.5-14 g, more preferably 4.0-13 g, still more preferably 4.5-12 g, even more preferably 5.0-12 g, further more preferably 5.5-12 g, even further more preferably 6.0-10 g, still even further more preferably 7.0-10 g and most preferably 8.0-10 g triglyceride fat"
- at least one additional edible ingredient, and
- water at most 15 %, preferably at most 10 %, and most preferably at most 5 % by weight of the portion.

5. The snack portion according to any one of claims 1 to 4, wherein the snack portion comprises
- 1.8-10 g, preferably 2.0-8.0 g total plant sterol and plant stanol equivalents, of which equivalents 50-100 %, preferably at least 70 % by weight are plant stanol equivalents, and of which equivalents
a) 0.20-2.0 g, preferably 0.22 - 1.3 g, more preferably 0.25-1.0 g are in free form, provided that 10-30 %, preferably 16-30 % and most preferably 20-30 % are in free form, and that 50-100 %, preferably at least 70 % by weight of the equivalents in free form are plant sterols, and
b) the rest of the equivalents are in esterified form
- 3.0-15 g, more preferably 4.0-13 g, still more preferably 5.0-12 g, further more preferably 6.0-10 g, still further more preferably 7.0-10 and most preferably 8.0-10 g triglyceride fat,
- at least one additional edible ingredient comprising 1.5 - 30 g, preferably 3.0-20 g, more preferably 4.0-15 g, even more preferably 8.0-15 g, and most preferably 9.0-15 g protein, and
- water at most 15 %, preferably at most 10 %, and most preferably at most 5 % by weight of the portion,
and the size of the snack portion is 10-100 g, preferably 20-60 g, more preferably 30-50 g.

6. A snack product with serum LDL cholesterol lowering effect, wherein the snack product comprises
- 1.0-20 w-%, preferably 1.2-15 w-%, more preferably 1.5-12 w-% and most preferably 2.0-10 w-% total plant sterol and plant stanol equivalents, of which equivalents by weight
a) 5.0-30 %, preferably 7.0-30 %, more preferably 8.0-30 %, still more preferably 10-30 %, even more preferably 13-30 %, further more preferably 16-30 %, most preferably 20-30 % are in free form, and
b) the rest of the equivalents are in esterified form,
- 2.0-30 w-%, preferably 4.0-28 w-%, more preferably 6.0-25 w-%, even more preferably 8.0-25 w-%, still more preferably 10-25 w-%, further more preferably 12-25 %, and even further more preferably 12-20 w-% and most preferably 15-20 w-% triglyceride fat,
- 0.1-15 w-%, preferably 0.1-10 w-%, and most preferably 0.1-5.0 w-% water, and
- 35-96.9 w-% at least one additional edible ingredient.

7. The snack product according to claim 6, wherein the amount of triglyceride fat is at least 3.0 g, preferably 3.0-15 g, more preferably 4.0-13 g, and most preferably 4.0-6.0 g per serving.

8. The snack product according to claim 6 or 7, wherein the additional edible ingredients comprise protein in an amount of 1.0 to 50 % by weight, preferably 3.0-45 %, more preferably 4.0-40 %, still more preferably 6.0-35 %, even more preferably 7.0-35 %, further more preferably 8.0-35 %, still even more preferably 10-30 %, still further more preferably 16-30 %, and most preferably 20-30 % by weight of the snack product.

9. The snack product according to any one of claims 6 to 8, wherein the snack product comprises
- 1.5-12 w-%, preferably 2.0-10 w-% total plant sterol and plant stanol equivalents, of which equivalents by weight
a) 10-30 %, preferably 13-30 %, more preferably 16-30 %, most preferably 20-30 % are in free form, and
b) the rest of the equivalents are in esterified form,
- 10-25 w-%, preferably 12-25 %, more preferably 12-20 w-% and most preferably 15-20 w-% triglyceride fat,
- 0.1-15 w-%, preferably 0.1-10 w-%, and most preferably 0.1-5.0 w-% water, and
- at least one additional edible ingredient as the rest of 100 w-%, the ingredient comprising 6.0-35 w-%, preferably 7.0-35 w-%, more preferably 8.0-35 w-%, still more preferably 10-30 w-%, further more preferably 16-30 w-% and most preferably 20-30 w-% protein of the snack product.

10. The snack product according to any one of claims 6 to 9, wherein the snack product comprises
- 1.5-12 w-%, preferably 2.0-10 w-% total plant sterol and plant stanol equivalents, of which equivalents 50-100 %, preferably at least 70 % by weight are plant stanol equivalents, and of which equivalents by weight
a) 10-30 %, preferably 13-30 %, more preferably 16-30 %, most preferably 20-30 % are in free form, provided that 50-100 %, preferably at least 70 % by weight of the equivalents in free form are plant sterols, and
b) the rest of the equivalents are in esterified form,
- 10-25 w-%, preferably 12-25 %, more preferably 12-20 w-% and most preferably 15-20 w-% triglyceride fat,
- 0.1-15 w-%, preferably 0.1-10 w-%, and most preferably 0.1-5.0 w-% water, and
- at least one additional edible ingredient as the rest of 100 w-%, the ingredient comprising 6.0-35 w-%, preferably 7.0-35 w-%, more preferably 8.0-35 w-%, still more preferably 10-30 w-%, further more preferably 16-30 w-% and most preferably 20-30 w-% protein of the snack product.

11. A pack containing the snack product according to any one of claims 6 to 10, or the snack portion according to any one of claims 1 to 5.

12. The snack portion according to any one of claims 1 to 5, the snack product according to any one of claims 6 to 10, or the pack according to claim 11, wherein the snack portion or snack product has an ensured serum LDL cholesterol lowering effect when used as a snack.

13. The snack portion according to any one of claims 1 to 5 or 12, the snack product according to any one of claims 6 to 10 or 12, or the pack according to claim 11 or 12, wherein the consumer is advised that the portion or product can be used as a snack.

14. The snack portion according to any one of claims 1 to 5, 12 or 13, the snack product according to any one of claims 6 to 10, 12 or 13, or the pack according to any one of claims 11 to 13, wherein the snack portion or snack product has at least 70 % of the serum LDL cholesterol lowering effect when consumed without a meal compared to when a same amount of total plant sterol and plant stanol equivalents is consumed with a meal.

15. The snack portion according to any one of claims 1 to 5 or 12 to 14, the snack product according to any one of claims 6 to 10 or 12 to 14, or the pack according to any one of claims 11 to 14, wherein the snack portion or snack product maintains the serum LDL cholesterol lowering efficacy regardless of the consumption time of the snack portion or snack product in relation to ingesting a meal.

16. The snack portion according to any one of claims 1 to 5 or 12 to 15, the snack product according to any one of claims 6 to 10 or 12 to 15, or the pack according to any one of claims 11 to 15, wherein the portion size of the snack product or the size of the snack portion is from 10 to 100 g, preferably from 20 to 60 g, and most preferably from 30 to 50 g.

17. The snack portion according to any one of claims 1 to 5 or 12 to 16, the snack product according to any one of claims 6 to 10 or 12 to 16, or the pack according to any one of claims 11 to 16, wherein the snack product or snack portion is selected from the group consisting of cookies, bars, crackers, wafers, buns, cakes, tortilla chips, bread sticks, candies, preferably cookies, bars and crackers, more preferably cookies and bars, and most preferably the snack portion or snack product are cookies.

18. The snack portion according to any one of claims 1-5 or 12-17 or the snack product according to any one of claims 6-10 or 12-17 for use in lowering serum LDL cholesterol, in a subject in need thereof, **characterised in that** the snack portion or the snack product is ingested without ingesting a meal.

## Patentansprüche

1. Snackportion mit Serum-LDL-Cholesterin-senkender Wirkung, wobei die Snackportion umfasst:
- 0,8-15 g, vorzugsweise 1,0-15 g, bevorzugter 1,5-12 g, noch bevorzugter 1,8-10 g, noch mehr bevorzugt 2,0-8,0 g, weiter bevorzugter 2,2-7,0 g und am bevorzugtesten 2,5-6,0 g Gesamt Pflanzensterol und Pflanzenstanol Äquivalente, von welchen Äquivalenten
a) 0,10-3,0 g, vorzugsweise 0,15-3,0 g, bevorzugter 0,18-2,5 g, noch bevorzugter 0,20-2,0 g, noch mehr bevorzugt 0,20-1,5 g, noch mehr bevorzugter 0,22-1,3 g und am bevorzugtesten 0,25-1,0 g in freier Form vorliegen, vorausgesetzt, dass 5,0-30 %, vorzugsweise 7,0-30 %, bevorzugter 8,0-30 %, noch bevorzugter 10-30 %, noch mehr bevorzugt 13-30 %, weiter bevorzugter 16-30 %, am bevorzugtesten 20-30 % in freier Form vorliegen, und
b) der Rest der Äquivalente in veresterter Form vorliegt,
- 3,0-15 g, vorzugsweise 3,5-14 g, bevorzugter 4,0-13 g, noch bevorzugter 4,5-12 g, noch mehr bevorzugt 5,0-12 g, weiter bevorzugter 5,5-12 g, noch mehr bevorzugter 6,0-10 g, weiter noch mehr bevorzugt 7,0-10 g und am bevorzugtesten 8,0-10 g Triglyceridfett,
- mindestens eine zusätzliche essbare Zutat und
- Wasser zu höchstens 15 Gew.-%, vorzugsweise höchstens 10 Gew.-% und am bevorzugtesten höchstens 5 Gew.-% der Portion.

2. Snackportion nach Anspruch 1, wobei die zusätzlichen essbaren Zutaten Protein in einer Menge von 1,5-30 g, bevorzugter 2,0-25 g, noch bevorzugter 3,0-20 g, noch mehr bevorzugt 4,0-15 g, weiter bevorzugter 8,0-15 g und am bevorzugtesten 9,0-15 g umfassen.

3. Snackportion nach Anspruch 1 oder 2, wobei sie umfasst:
- 1,8-10 g, vorzugsweise 2,0-8,0 g Gesamt Pflanzensterol und Pflanzenstanol Äquivalente, von welchen Äquivalenten
a) 0,20-2,0 g, vorzugsweise 0,22-1,3 g, bevorzugter 0,25-1,0 g in freier Form vorliegen, vorausgesetzt, dass 10-30 %, vorzugsweise 13-30 %, bevorzugter 16-30 % und am bevorzugtesten 20-30 % in freier Form vorliegen, und
b) der Rest der Äquivalente in veresterter Form vorliegt,
- 4,0-13 g, bevorzugter 5,0-12 g, noch bevorzugter 6,0-10 g, weiter bevorzugter 7,0-10 g und am bevorzugtesten 8,0-10 g Triglyceridfett,
- mindestens eine zusätzliche essbare Zutat, umfassend 1,5-30 g, vorzugsweise 3,0-20 g, bevorzugter 4,0-15 g, noch mehr bevorzugt 8,0-15 g und am bevorzugtesten 9,0-15 g Protein, und
- Wasser zu höchstens 15 Gew.-%, vorzugsweise höchstens 10 Gew.-% und am bevorzugtesten höchstens 5 Gew.-% der Portion,
und die Größe der Snackportion 10-100 g, vorzugsweise 20-60 g, bevorzugter 30-50 g beträgt.

4. Snackportion nach Anspruch 1, wobei die Snackportion umfasst:
- 0,8-15 g, vorzugsweise 1,0-15 g, bevorzugter 1,5-12 g, noch bevorzugter 1,8-10 g, noch mehr bevorzugt 2,0-8,0 g, weiter bevorzugter 2,2-7,0 g und am bevorzugtesten 2,5-6,0 g Gesamt Pflanzensterol und Pflanzenstanol Äquivalente, von welchen Äquivalenten 50-100 Gew.-%, vorzugsweise mindestens 70 Gew.-% Pflanzenstanol Äquivalente sind, und von welchen Äquivalenten
a) 0,10-3,0 g, vorzugsweise 0,15-3,0 g, bevorzugter 0,18-2,5 g, noch bevorzugter 0,20-2,0 g, noch mehr bevorzugt 0,20-1,5 g, noch mehr bevorzugter 0,22-1,3 g und am bevorzugtesten 0,25-1,0 g in freier Form vorliegen, vorausgesetzt, dass 5,0-30 %, vorzugsweise 7,0-30 %, bevorzugter 8,0-30 %, noch bevorzugter 10-30 %, noch mehr bevorzugt 13-30 %, weiter bevorzugter 16-30 % und am bevorzugtesten 20-30 % in freier Form vorliegen, und 50-100 Gew.-%, vorzugsweise mindestens 70 Gew.-% der Äquivalente in freier Form Pflanzensterole sind, und
b) der Rest der Äquivalente in veresterter Form vorliegt,
- 3,0-15 g, bevorzugt 3,5-14 g, bevorzugter 4,0-13 g, noch bevorzugter 4,5-12 g, noch mehr bevorzugt 5,0-12 g, weiter bevorzugter 5,5-12 g, noch weiter bevorzugter 6,0-10 g, sogarnoch weiter bevorzugter 7,0-10 g und am bevorzugtesten 8,0-10 g Triglyceridfett,
- mindestens eine zusätzliche essbare Zutat und
- Wasser zu höchstens 15 Gew.-%, vorzugsweise höchstens 10 Gew.-% und am bevorzugtesten höchstens 5 Gew.-% der Portion.

5. Snackportion nach einem der Ansprüche 1 bis 4, wobei die Snackportion umfasst:
- 1,8-10 g, vorzugsweise 2,0-8,0 g Gesamt Pflanzensterol und Pflanzenstanol Äquivalente, von welchen Äquivalenten 50-100 Gew.-%, vorzugsweise mindestens 70 Gew.-% Pflanzenstanol Äquivalente sind, und von welchen Äquivalenten
a) 0,20-2,0 g, vorzugsweise 0,22-1,3 g, bevorzugter 0,25-1,0 g in freier Form vorliegen, vorausgesetzt, dass 10-30 %, vorzugsweise 16-30 % und am bevorzugtesten 20-30 % in freier Form vorliegen und 50-100 Gew.-%, vorzugsweise mindestens 70 Gew.-% der Äquivalente in freier Form Pflanzensterole sind, und
b) der Rest der Äquivalente in veresterter Form vorliegt,
- 3,0-15 g, bevorzugter 4,0-13 g, noch bevorzugter 5,0-12 g, weiter bevorzugter 6,0-10 g, noch weiter bevorzugter 7,0-10 und am bevorzugtesten 8,0-10 g Triglyceridfett,
- mindestens eine zusätzliche essbare Zutat, umfassend 1,5-30 g, vorzugsweise 3,0-20 g, bevorzugter 4,0-15 g, noch bevorzugter 8,0-15 g und am bevorzugtesten 9,0-15 g Protein, und
- Wasser zu höchstens 15 Gew.-%, vorzugsweise höchstens 10 Gew.-% und am bevorzugtesten höchstens 5 Gew.-% der Portion,
und die Größe der Snackportion 10-100 g, vorzugsweise 20-60 g, bevorzugter 30-50 g beträgt.

6. Snackprodukt mit Serum-LDL-Cholesterin-senkender Wirkung, wobei das Snackprodukt umfasst:
- 1,0-20 Gew.-%, vorzugsweise 1,2-15 Gew.-%, bevorzugter 1,5-12 Gew.-% und am bevorzugtesten 2,0-10 Gew.-% Gesamt Pflanzensterol und Pflanzenstanol Äquivalente, von welchen Äquivalenten in Gewichtsprozent
a) 5,0-30 %, vorzugsweise 7,0-30 %, bevorzugter 8,0-30 %, noch bevorzugter 10-30 %, noch mehr bevorzugt 13-30 %, weiter bevorzugter 16-30 %, am bevorzugtesten 20-30 % in freier Form vorliegen und
b) der Rest der Äquivalente in veresterter Form vorliegt,
- 2,0-30 Gew.-%, vorzugsweise 4,0-28 Gew.-%, bevorzugter 6,0-25 Gew.-%, noch mehr bevorzugt 8,0-25 Gew.-%, noch bevorzugter 10-25 Gew.-%, weiter bevorzugter 12-25 % und noch weiter bevorzugter 12-20 Gew.-% und am meisten bevorzugt 15-20 Gew.-% Triglyceridfett,
- 0,1-15 Gew.-%, vorzugsweise 0,1-10 Gew.-% und am bevorzugtesten 0,1-5,0 Gew.-% Wasser und
- 35-96,9 Gew.-% mindestens eine zusätzliche essbare Zutat.

7. Snackprodukt nach Anspruch 6, wobei die Menge an Triglyceridfett mindestens 3,0 g, vorzugsweise 3,0-15 g, bevorzugter 4,0-13 g und am meisten bevorzugt 4,0-6,0 g pro Ration beträgt.

8. Snackprodukt nach Anspruch 6 oder 7, wobei die zusätzlichen essbaren Zutaten Protein in einer Menge von 1,0 bis 50 Gew.-%, vorzugsweise 3,0-45 Gew.-%, bevorzugter 4,0-40 Gew.-%, noch bevorzugter 6,0-35 Gew.-%, noch mehr bevorzugt 7,0-35 Gew.-%, weiter bevorzugter 8,0-35 Gew.-%, noch bevorzugter 10-30 Gew.-%, noch weiter bevorzugter 16-30 Gew.-% und am bevorzugtesten 20-30 Gew.-% des Snackprodukts umfassen.

9. Snackprodukt nach einem der Ansprüche 6 bis 8, wobei das Snackprodukt umfasst:
- 1,5-12 Gew.-%, vorzugsweise 2,0-10 Gew.-% Gesamt Pflanzensterol und Pflanzenstanol Äquivalente, von welchen Äquivalenten in Gewichtsprozent
a) 10-30 %, vorzugsweise 13-30 %, bevorzugter 16-30 %, am bevorzugtesten 20-30 % in freier Form vorliegen und
b) der Rest der Äquivalente in veresterter Form vorliegt,
- 10-25 Gew.-%, vorzugsweise 12-25 %, bevorzugter 12-20 Gew.-% und am bevorzugtesten 15-20 Gew.-% Triglyceridfett,
- 0,1-15 Gew.-%, vorzugsweise 0,1-10 Gew.-% und am bevorzugtesten 0,1-5,0 Gew.-% Wasser und
- mindestens eine zusätzliche essbare Zutat als Rest von 100 Gew.-%, wobei die Zutat 6,0-35 Gew.-%, vorzugsweise 7,0-35 Gew.-%, bevorzugter 8,0-35 Gew.-%, noch bevorzugter 10-30 Gew.-%, weiter bevorzugter 16-30 Gew.-% und am bevorzugtesten 20-30 Gew.-% Protein des Snackprodukts umfasst.

10. Snackprodukt nach einem der Ansprüche 6 bis 9, wobei das Snackprodukt umfasst:
- 1,5-12 Gew.-%, vorzugsweise 2,0-10 Gew.-% Gesamt Pflanzensterol und Pflanzenstanol Äquivalente, von welchen Äquivalenten 50-100 Gew.-%, vorzugsweise mindestens 70 Gew.-% Pflanzenstanol Äquivalente sind, und von welchen Äquivalenten in Gewichtsprozent
a) 10-30 %, vorzugsweise 13-30 %, bevorzugter 16-30 %, am bevorzugtesten 20-30 % in freier Form vorliegen, vorausgesetzt, dass 50-100 Gew.-%, vorzugsweise mindestens 70 Gew.-% der Äquivalente in freier Form Pflanzensterole sind, und
b) der Rest der Äquivalente in veresterter Form vorliegt,
- 10-25 Gew.-%, vorzugsweise 12-25 %, bevorzugter 12-20 Gew.-% und am bevorzugtesten 15-20 Gew.-% Triglyceridfett,
- 0,1-15 Gew.-%, vorzugsweise 0,1-10 Gew.-% und am bevorzugtesten 0,1-5,0 Gew.-% Wasser und
- mindestens eine zusätzliche essbare Zutat als Rest von 100 Gew.-%, wobei die Zutat 6,0-35 Gew.-%, vorzugsweise 7,0-35 Gew.-%, bevorzugter 8,0-35 Gew.-%, noch bevorzugter 10-30 Gew.-%, weiter bevorzugter 16-30 Gew.-% und am bevorzugtesten 20-30 Gew.-% Protein des Snackprodukts umfasst.

11. Packung, die das Snackprodukt nach einem der Ansprüche 6 bis 10 oder die Snackportion nach einem der Ansprüche 1 bis 5 enthält.

12. Snackportion nach einem der Ansprüche 1 bis 5, Snackprodukt nach einem der Ansprüche 6 bis 10 oder Packung nach Anspruch 11, wobei die Snackportion oder das Snackprodukt eine gewährleistete Serum-LDL-Cholesterin-senkende Wirkung aufweist, wenn sie/es als Snack verwendet wird.

13. Snackportion nach einem der Ansprüche 1 bis 5 oder 12, Snackprodukt nach einem der Ansprüche 6 bis 10 oder 12 oder Packung nach Anspruch 11 oder 12, wobei der Verbraucher darauf hingewiesen wird, dass die Portion oder das Produkt als Snack verwendet werden kann.

14. Snackportion nach einem der Ansprüche 1 bis 5, 12 oder 13, Snackprodukt nach einem der Ansprüche 6 bis 10, 12 oder 13 oder Packung nach einem der Ansprüche 11 bis 13, wobei die Snackportion oder das Snackprodukt mindestens 70 % der Serum-LDL-Cholesterin-senkenden Wirkung aufweist, wenn sie/es ohne Mahlzeit verzehrt wird, verglichen damit, wenn die gleiche Menge an Gesamt Pflanzensterol und Pflanzenstanol Äquivalenten mit einer Mahlzeit verzehrt wird.

15. Snackportion nach einem der Ansprüche 1 bis 5 oder 12 bis 14, Snackprodukt nach einem der Ansprüche 6 bis 10 oder 12 bis 14 oder Packung nach einem der Ansprüche 11 bis 14, wobei die Snackportion oder das Snackprodukt die Serum-LDL-Cholesterin-senkende Wirkung unabhängig von der Verzehrzeit der Snackportion oder des Snackprodukts in Bezug auf die Einnahme einer Mahlzeit aufrechterhält.

16. Snackportion nach einem der Ansprüche 1 bis 5 oder 12 bis 15, Snackprodukt nach einem der Ansprüche 6 bis 10 oder 12 bis 15 oder Packung nach einem der Ansprüche 11 bis 15, wobei die Portionsgröße des Snackprodukts oder die Größe der Snackportion 10 bis 100 g, vorzugsweise 20 bis 60 g und am bevorzugtesten 30 bis 50 g beträgt.

17. Snackportion nach einem der Ansprüche 1 bis 5 oder 12 bis 16, Snackprodukt nach einem der Ansprüche 6 bis 10 oder 12 bis 16 oder Packung nach einem der Ansprüche 11 bis 16, wobei das Snackprodukt oder die Snackportion aus der Gruppe, bestehend aus Keksen, Riegeln, Crackern, Waffeln, Brötchen, Kuchen, Tortillachips, Brotstangen, Süßigkeiten, vorzugsweise Keksen, Riegeln und Crackern, bevorzugter Keksen und Riegeln ausgewählt ist und am bevorzugtesten die Snackportion oder das Snackprodukt Kekse sind.

18. Snackportion nach einem der Ansprüche 1 - 5 oder 12 - 17 oder Snackprodukt nach einem der Ansprüche 6 - 10 oder 12 - 17 zur Verwendung bei der Senkung des Serum-LDL-Cholesterins bei einem Probanden, der dies benötigt, **dadurch gekennzeichnet, dass** die Snackportion oder das Snackprodukt ohne Einnahme einer Mahlzeit eingenommen wird.

## Revendications

1. Portion de grignotage ayant un effet abaissant le cholestérol LDL sérique, la portion de grignotage comprenant
- 0,8 à 15 g, de préférence 1,0 à 15 g, plus préférablement 1,5 à 12 g, encore plus préférablement 1,8 à 10 g, encore plus préférablement 2,0 à 8,0 g, encore plus préférablement 2,2 à 7,0 g, et idéalement 2,5 à 6,0 g d'équivalents totaux de stérols végétaux et de stanols végétaux, desquels équivalents
a) 0,10 à 3,0 g, de préférence 0,15 à 3,0 g, plus préférablement 0,18 à 2,5 g, encore plus préférablement 0,20 à 2,0 g, encore plus préférablement 0,20 à 1,5 g, encore plus préférablement 0,22 à 1,3 g, et idéalement 0,25 à 1,0 g sont sous forme libre, à condition que 5,0 à 30 %, de préférence 7,0 à 30 %, plus préférablement 8,0 à 30 %, encore plus préférablement 10 à 30 %, encore plus préférablement 13 à 30 %, encore plus préférablement 16 à 30 %, idéalement 20 à 30 % soient sous forme libre, et
b) le reste des équivalents est sous forme estérifiée,
- 3,0 à 15 g, de préférence 3,5 à 14 g, plus préférablement 4,0 à 13 g, encore plus préférablement 4,5 à 12 g, encore plus préférablement 5,0 à 12 g, encore plus préférablement 5,5 à 12 g, encore plus préférablement 6,0 à 10 g, encore plus préférablement 7,0 à 10 g, et idéalement 8,0 à 10 g de lipides triglycérides,
- au moins un ingrédient comestible supplémentaire, et
- de l'eau à au plus 15 %, de préférence à au plus 10 %, et idéalement à au plus 5 % en poids de la portion.

2. Portion de grignotage selon la revendication 1, dans laquelle les ingrédients comestibles supplémentaires comprennent des protéines en une quantité de 1,5 à 30 g, plus préférablement de 2,0 à 25 g, encore plus préférablement de 3,0 à 20 g, encore plus préférablement de 4,0 à 15 g, encore plus préférablement de 8,0 à 15 g, et idéalement de 9,0 à 15 g.

3. Portion de grignotage selon la revendication 1 ou 2 comprenant
- 1,8 à 10 g, de préférence 2,0 à 8,0 g d'équivalents totaux de stérols végétaux et de stanols végétaux, desquels équivalents
a) 0,20 à 2,0 g, de préférence 0,22 à 1,3 g, plus préférablement 0,25 à 1,0 g sont sous forme libre, à condition que 10 à 30 %, de préférence 13 à 30 %, plus préférablement 16 à 30 % et idéalement 20 à 30 % soient sous forme libre, et
b) le reste des équivalents est sous forme estérifiée,
- 4,0 à 13 g, plus préférablement 5,0 à 12 g, encore plus préférablement 6,0 à 10 g, encore plus préférablement 7,0 à 10 g et idéalement 8,0 à 10 g de lipides triglycérides,
- au moins un ingrédient comestible supplémentaire comprenant 1,5 à 30 g, de préférence 3,0 à 20 g, plus préférablement 4,0 à 15 g, encore plus préférablement 8,0 à 15 g et idéalement 9,0 à 15 g de protéines, et
- de l'eau à au plus 15 %, de préférence à au plus 10 %, et idéalement à au plus 5 % en poids de la portion,
et la taille de la portion de grignotage est de 10 à 100 g, de préférence de 20 à 60 g, idéalement de 30 à 50 g.

4. Portion de grignotage selon la revendication 1, la portion de grignotage comprenant
- 0,8 à 15 g, de préférence 1,0 à 15 g, plus préférablement 1,5 à 12 g, encore plus préférablement 1,8 à 10 g, encore plus préférablement 2,0-8,0 g, encore plus préférablement 2,2-7,0 g, et idéalement 2,5 à 6,0 g d'équivalents totaux de stérols végétaux et de stanols végétaux, desquels équivalents 50 à 100 %, de préférence au moins 70 % en poids sont des équivalents de stanols végétaux, et desquels équivalents
a) 0,10 à 3,0 g, de préférence 0,15 à 3,0 g, plus préférablement 0,18 à 2,5 g, encore plus préférablement 0,20 à 2,0 g, encore plus préférablement 0,20 à 1,5 g, encore plus préférablement 0,22 à 1,3 g, et idéalement 0,25 à 1,0 g sont sous forme libre, à condition que 5,0 à 30 %, de préférence 7,0 à 30 %, plus préférablement 8,0 à 30 %, encore plus préférablement 10 à 30 %, encore plus préférablement 13 à 30 %, encore plus préférablement 16 à 30 %, idéalement 20 à 30 % soient sous forme libre, et que 50 à 100 %, de préférence au moins 70 % en poids des équivalents sous forme libre soient des stérols végétaux, et
b) le reste des équivalents est sous forme estérifiée,
- 3,0 à 15 g, de préférence 3,5 à 14 g, plus préférablement 4,0 à 13 g, encore plus préférablement 4,5 à 12 g, encore plus préférablement 5,0 à 12 g, encore plus préférablement 5,5 à 12 g, encore plus préférablement 6,0 à 10 g, encore plus préférablement 7,0 à 10 g, et idéalement 8,0 à 10 g de lipides triglycérides,
- au moins un ingrédient comestible supplémentaire, et
- de l'eau à au plus 15 %, de préférence à au plus 10 %, et idéalement à au plus 5 % en poids de la portion.

5. Portion de grignotage selon l'une quelconque des revendications 1 à 4, dans laquelle la portion de grignotage comprend
- 1,8 à 10 g, de préférence 2,0 à 8,0 g d'équivalents totaux de stérols végétaux et de stanols végétaux, desquels équivalents 50 à 100 %, de préférence au moins 70 % en poids sont des équivalents de stanols végétaux, et desquels équivalents
a) 0,20 à 2,0 g, de préférence 0,22 à 1,3 g, plus préférablement 0,25 à 1,0 g, sont sous forme libre, à condition que 10 à 30 %, de préférence 16 à 30 % et idéalement 20 à 30 % soient sous forme libre, et que 50 à 100 %, de préférence au moins 70 % en poids des équivalents sous forme libre soient des stérols végétaux, et
b) le reste des équivalents est sous forme estérifiée
- 3,0 à 15 g, plus préférablement 4,0 à 13 g, encore plus préférablement 5,0 à 12 g, encore plus préférablement 6,0 à 10 g, encore plus préférablement 7,0 à 10 et idéalement 8,0 à 10 g de lipides triglycérides,
- au moins un ingrédient comestible supplémentaire comprenant 1,5 à 30 g, de préférence 3,0 à 20 g, plus préférablement 4,0 à 15 g, encore plus préférablement 8,0 à 15 g, et idéalement 9,0 à 15 g de protéines, et
- de l'eau à au plus 15 %, de préférence à au plus 10 %, et idéalement à au plus 5 % en poids de la portion,
et la taille de la portion de grignotage est de 10 à 100 g, de préférence de 20 à 60 g, plus préférablement de 30 à 50 g.

6. Produit de grignotage ayant un effet d'abaissement du cholestérol LDL sérique, le produit de grignotage comprenant
- 1,0 à 20 % en poids, de préférence 1,2 à 15 % en poids, plus préférablement 1,5 à 12 % en poids et idéalement 2,0 à 10 % en poids d'équivalents totaux de stérols végétaux et de stanols végétaux, desquels équivalents en poids
a) 5,0 à 30 %, de préférence 7,0 à 30 %, plus préférablement 8,0 à 30 %, encore plus préférablement 10 à 30 %, encore plus préférablement 13 à 30 %, encore plus préférablement 16 à 30 %, idéalement 20 à 30 % sont sous forme libre, et
b) le reste des équivalents est sous forme estérifiée,
- 2,0 à 30 % en poids, de préférence 4,0 à 28 % en poids, plus préférablement 6,0 à 25 % en poids, encore plus préférablement 8,0 à 25 % en poids, encore plus préférablement 10 à 25 % en poids, encore plus préférablement 12- 25 %, et encore plus préférablement 12 à 20 % en poids et idéalement 15 à 20 % en poids de lipides triglycérides,
- 0,1 à 15 % en poids, de préférence 0,1 à 10 % en poids, et idéalement 0,1 à 5,0 % en poids d'eau, et
- 35 à 96,9 % en poids d'au moins un ingrédient comestible supplémentaire.

7. Produit de grignotage selon la revendication 6, dans lequel la quantité de lipides triglycérides est d'au moins 3,0 g, de préférence de 3,0 à 15 g, plus préférablement de 4,0 à 13 g, et idéalement de 4,0 à 6,0 g par portion.

8. Produit de grignotage selon la revendication 6 ou 7, dans lequel les ingrédients comestibles supplémentaires comprennent des protéines en une quantité de 1,0 à 50 % en poids, de préférence de 3,0 à 45 %, plus préférablement de 4,0 à 40 %, encore plus préférablement de 6,0 à 35 %, encore plus préférablement 7,0 à 35 %, encore plus préférablement 8,0 à 35 %, encore plus préférablement 10 à 30 %, encore plus préférablement 16 à 30 %, et idéalement 20 à 30 % en poids du produit de grignotage.

9. Produit de grignotage selon l'une quelconque des revendications 6 à 8, dans lequel le produit de grignotage comprend
- 1,5 à 12 % en poids, de préférence 2,0 à 10 % en poids d'équivalents totaux de stérols végétaux et de stanols végétaux, desquels équivalents en poids
a) 10 à 30 %, de préférence 13 à 30 %, plus préférablement 16 à 30 %, idéalement 20 à 30 % sont sous forme libre, et
b) le reste des équivalents est sous forme estérifiée,
- 10 à 25 % en poids, de préférence 12 à 25 %, plus préférablement 12 à 20 % en poids et idéalement 15 à 20 % en poids de lipides triglycérides,
- 0,1 à 15 % en poids, de préférence 0,1 à 10 % en poids, et idéalement 0,1 à 5,0 % en poids d'eau, et
- au moins un ingrédient comestible supplémentaire pour arriver à 100 % en poids, l'ingrédient comprenant 6,0 à 35 % en poids, de préférence 7,0 à 35 % en poids, plus préférablement 8,0 à 35 % en poids, encore plus préférablement 10 à 30 % en poids, encore plus préférablement 16 à 30 % en poids et idéalement 20 à 30 % en poids des protéines du produit de grignotage.

10. Produit de grignotage selon l'une quelconque des revendications 6 à 9, dans lequel le produit de grignotage comprend
- 1,5 à 12 % en poids, de préférence 2,0 à 10 % en poids d'équivalents totaux de stérols végétaux et de stanols végétaux, desquels équivalents 50 à 100 %, de préférence au moins 70 % en poids sont des équivalents de stanols végétaux, et desquels équivalents en poids
a) 10 à 30 %, de préférence 13 à 30 %, plus préférablement 16 à 30 %, idéalement 20 à 30 % sont sous forme libre, à condition que 50 à 100 %, de préférence au moins 70 % en poids des équivalents sous forme libre soient des stérols végétaux, et
b) le reste des équivalents est sous forme estérifiée,
- 10 à 25 % en poids, de préférence 12 à 25 %, plus préférablement 12 à 20 % en poids et idéalement 15 à 20 % en poids de lipides triglycérides,
- 0,1 à 15 % en poids, de préférence 0,1 à 10 % en poids, et idéalement 0,1 à 5,0 % en poids d'eau, et
- au moins un ingrédient comestible supplémentaire pour arriver à 100 % en poids, l'ingrédient comprenant 6,0 à 35 % en poids, de préférence 7,0 à 35 % en poids, plus préférablement 8,0 à 35 % en poids, encore plus préférablement 10 à 30 % en poids, encore plus préférablement 16 à 30 % en poids et idéalement 20 à 30 % en poids des protéines du produit de grignotage.

11. Emballage contenant le produit de grignotage selon l'une quelconque des revendications 6 à 10 ou la portion de grignotage selon l'une quelconque des revendications 1 à 5.

12. Portion de grignotage selon l'une quelconque des revendications 1 à 5, produit de grignotage selon l'une quelconque des revendications 6 à 10, ou emballage selon la revendication 11, dans lesquels la portion de grignotage ou le produit de grignotage ont un effet d'abaissement du cholestérol LDL sérique garanti lors d'une utilisation comme grignotage.

13. Portion de grignotage selon l'une quelconque des revendications 1 à 5 ou 12, produit de grignotage selon l'une quelconque des revendications 6 à 10 ou 12, ou emballage selon la revendication 11 ou 12, dans lequel le consommateur est informé que la portion ou le produit peuvent être utilisés comme grignotage.

14. Portion de grignotage selon l'une quelconque des revendications 1 à 5, 12 ou 13, produit de grignotage selon l'une quelconque des revendications 6 à 10, 12 ou 13, ou emballage selon l'une quelconque des revendications 11 à 13, dans lequel la portion de grignotage ou le produit de grignotage ont au moins 70 % de l'effet d'abaissement du cholestérol LDL sérique lorsqu'ils sont consommés sans repas par rapport au cas où une quantité identique d'équivalents totaux de stérols végétaux et de stanols végétaux est consommée avec un repas.

15. Portion de grignotage selon l'une quelconque des revendications 1 à 5 ou 12 à 14, produit de grignotage selon l'une quelconque des revendications 6 à 10 ou 12 à 14, ou emballage selon l'une quelconque des revendications 11 à 14, dans lequel la portion de grignotage ou le produit de grignotage conservent l'efficacité d'abaissement du cholestérol LDL sérique quel que soit le moment de la consommation de la portion de grignotage ou du produit de grignotage par rapport à l'ingestion d'un repas.

16. Portion de grignotage selon l'une quelconque des revendications 1 à 5 ou 12 à 15, produit de grignotage selon l'une quelconque des revendications 6 à 10 ou 12 à 15, ou emballage selon l'une quelconque des revendications 11 à 15, dans lequel la taille de la portion du produit de grignotage ou la taille de la portion de grignotage est de 10 à 100 g, de préférence de 20 à 60 g, et idéalement de 30 à 50 g.

17. Portion de grignotage selon l'une quelconque des revendications 1 à 5 ou 12 à 16, produit de grignotage selon l'une quelconque des revendications 6 à 10 ou 12 à 16, ou emballage selon l'une quelconque des revendications 11 à 16, dans lequel le produit de grignotage ou la portion de grignotage sont choisis dans le groupe comprenant les biscuits, les barres, les crackers, les gaufrettes, les brioches, les gâteaux, les chips de tortilla, les bâtonnets de pain, les bonbons, de préférence les biscuits, les barres et les crackers, plus préférablement les biscuits et les barres, et idéalement la portion de grignotage ou le produit de grignotage sont des biscuits.

18. Portion de grignotage selon l'une quelconque des revendications 1 à 5 ou 12 à 17 ou produit de grignotage selon l'une quelconque des revendications 6 à 10 ou 12 à 17 servant à réduire le cholestérol LDL sérique, chez un sujet en ayant besoin, **caractérisé en ce que** la portion de grignotage ou le produit de grignotage sont ingérés sans ingérer de repas.
